# EUROPEAN PATENT SPECIFICATION

(11) **EP 3 996 761 B1**
(45) Date of publication and mention of the grant of the patent: **05.08.2026**
(21) Application number: 20742428.4
(22) Date of filing: 29.05.2020
(51) Int. Cl.: A61L 27/36, A61L 27/50, A61L 27/52, A61L 27/46

(54) **ENHANCED OSTEOGENIC COMPOSITION**
VERBESSERTE OSTEOGENE ZUSAMMENSETZUNG
COMPOSITION OSTÉOGÉNIQUE AMÉLIORÉE

(30) Priority: 10.07.2019 US 201962872364 P
(43) Date of publication of application: 18.05.2022
(73) Proprietor: Access2Bone IP B.V., 2333 BD Leiden (NL)
(72) Inventor: SICKLER, Michael F., San Juan Capistrano, CA 92875 (US); VAN DE MORTEL, Arnoldus C.m., 233 BD Leiden (NL); DE ROODE, Nienke, 233 BD Leiden (NL)
(74) Representative: Nederlandsch Octrooibureau
(86) International application number: PCT/IB2020/000427
(87) International publication number: WO 2021/005412

(56) References cited:
- EP-A1- 0 495 284
- US-A1- 2018 104 382

## Description

### CLAIM OF BENEFIT OF FILING DATE

This application claims the benefit of the filing date of U.S. Provisional Application Serial No. 62/872364 titled: "Enhanced Osteogenic Composition" filed on July 10, 2019.

### FIELD OF INVENTION

This invention relates to a material exhibiting osteogenic activity and, more particularly, an enhanced osteogenic composition derived from demineralized bone capable of being mixed with a natural or synthetic implant and method of making same. The invention further relates to a natural or synthetic implant, e.g., allograft bone tissue and/or calcium phosphate which is mixed with or otherwise contains the enhanced osteogenic composition.

### BRIEF DESCRIPTION OF THE DRAWINGS

FIG. 1 is a photograph (100 times magnification) of a section of animal living tissue treated with the Enhanced Osteogenic Composition of the present invention and demineralized bone material as described below in Example 14; and
FIG. 2 is a photograph (100 times magnification) of a section of animal living tissue treated with demineralized bone material only as described below in Example 14.

### DESCRIPTION OF THE PREFERRED EMBODIMENT

The use of osteogenic composition derived from demineralized cortical cancellous and/or corticocancellous autogenic, allogeneic or xenogeneic bone (hereinafter referred to as "Bone") in surgical bone repairs and/or reconstruction has been known for decades. The conventional production of such osteogenic composition begins with pulverization of the Bone resulting in Bone particles generally ranging from 100 microns to 300 microns in average particle size. Thereafter, these pulverized Bone particles are defatted and disinfected using a suitable solvent (e.g., ethanol or the like). Once the pulverized Bone particles have been defatted and disinfected, they are immersed in acid over time to effect demineralization. The demineralization process removes essentially all of the original calcium content of the Bone resulting in a demineralized bone material containing at least less than about 10% weight calcium with the preferred range from about 0.5% to 1% weight percent calcium. The term "about" used herein this specification is defined as plus or minus 1%. The demineralized Bone particles are then subject to an acid-promoted cleavage process using acid in the concentration of 2M to 3M for a few minutes to several hours either in ambient temperature (e.g., below about 25° C) or a heated environment to a temperature of about 30° C to about 75° C, resulting in a phase change from solid to a viscous liquid of osteogenic composition. This liquid osteogenic composition can then be further processed via titration, dialysis, and lyophilization into dry powder for easy handling and storage. See e.g., U.S. Patent No. 5,236,456; Section E Processing and Storage, Standards for Tissue Banking, 14th Edition (2016), American Association of Tissue Banks.

US 2018/104382 A1 discloses a method of providing an osteoinductive composition by extracting 2-10 g of DBM with 100 g of a 3-6M solution of an organic acid (citric, acetic, lactic, or malic) for 24-96 h at a temperature not greater than 50°C. The solution is then separated by the solids by filtration or ultrafiltration, in particular dialysis with a cut off from 10 to 12 kDa performed after the pH is brought to 6.5-7.5. The DBM extract is lyophilized onto a structure.

The present invention provides an osteogenic composition exhibiting enhanced and/or improved osteogenic activity (hereinafter referred to as "enhanced osteogenic composition" or "EOC") when compared to currently commercially available osteogenic compositions (e.g., the ones described in U.S. Patent No. 5,236,456). EOC's enhanced and/or improved osteogenic activity is highly desired for use in surgical bone repairs and/or reconstruction. The present invention further provides a method of making the EOC as described below.

### I. Method of Making Enhanced Osteogenic Composition

In one exemplary embodiment of the present invention, EOC is prepared by a method according to claim 1.
It is preferred that the demineralized bone material shall contain no greater than about 10, no greater than about 1 and most preferably no greater than about 0.5 weight percent calcium.

Acids which can be used in the demineralization step include inorganic acids such as hydrochloric acid and organic acids such as peracetic acid. The concentration of the acid in this demineralization step is preferred to be about 0.5M to about 1.0M but other art-disclosed concentration(s) may be used. The amount of time for such acid immersion is preferred to be from about 20 minutes to about 2 hours under ambient conditions. However, acid immersion time is not limited to this range and may be shorter or longer as long as essentially all of the original calcium content of the Bone is removed during this demineralization step, resulting in a demineralized bone material ("DBM") containing at least less than about 10% weight calcium with the preferred range from about 0.5% to 1% weight percent calcium.

After the demineralization step, the method of the present invention further includes subjecting the DBM to an acid-promoted cleavage step (i.e., treating the DBM in an acidic extraction medium) performed at a temperature controlled environment (i.e., extraction temperature) of greater than 25°C but less than 30°C, preferably from 26°C to 29°C. The temperature ranges of between 26°C to 37°C, between 26°C to 35°C, between 26°C to 50°C, and between 26°C to 80°C are also included in the present invention.

While the acid-promoted cleavage step can utilize the same acid used in the demineralization step, it is preferred that an organic acid such as Ascorbic, Malic, Citric, Tartaric and/or combination thereof is used for the acid-promoted cleavage step in a concentration of at least about 3.0M to about 5.0M, preferably about 3.3M to about 4.4M, most preferable about 3.5M to about 4.2M. The preferred pH during this acid-promoted cleavage step is between 0.10 to 0.45, more preferably between 0.3 to 0.45, and most preferable between 0.35 to 0.45. Depending on the desired molarity, the acid treatment can have a ratio of acid to DBM at least about 1 ml/g to about 100ml/g, preferably about 5ml/g to 50ml/g, most preferable about 10mL/g to about 20mL/g. The acid treatment time can vary depending on average particle size(s) of the DBM and the temperature. Preferably the acid treatment time is from about 48 hours to about 120 hours, being sufficient to yield the desired results. The acid treatment time from about 48 hours to about 120 hours are also included in the present invention.

It should be noted that as an alternative, not claimed, embodiment, the demineralization step and the acid-promoted cleavage step can be carried out simultaneously (i.e., not separately) using the same acid(s), temperature(s), and pH(s).

The acid-promoted cleavage step yields a viscous liquid composition, which still retains a high concentration of organic acid so it can be titrated/diluted using a suitable basic substance and/or liquid and known techniques until a pH of about 3 is obtained. At this pH, the hydraulic degradation of the proteins caused by the acid is stopped.

Alternatively and/or additionally, the viscous liquid composition can be subjected to a dialysis step using a Regenerated Cellulose Membrane filter between 1.0 kilodaltons (kDa) to 12.0 kDa, preferably greater or equal to 3.5kDa, more preferably between 3.5 kDa to 6.0 kDa, and most preferably 3.5 kDa to 4 kDa. In addition to using deionized water alone, a buffer solution of calcium carbonate and/or calcium hydroxide can be used for dialysis to drive pH to a neutral solution. Dialysis using a Regenerated Cellulose Membrane filter provides a molecular separation for desalting and isolating proteins. After the diluting step and/or the dialysis step, EOC of the present invention is provided. During storage, at room or above freezing temperatures, the EOC remains in viscous liquid form having a viscosity such as about 10 to 16,000 centipoise. The molecular weight of the EOC is equal or greater than 3.5kDa. The EOC will remain in viscous liquid form if it is maintained in a sealed container at room temperature.

Alternatively, the EOC can be lyophilized using known techniques to yield a dry product. Thereafter, the lyophilized product (i.e., dry EOC) can be hydrated, as needed, into a suitable form for mixing or injection into desired treatment sites such as tissue, implants, or the like.

The EOC, whether dry, liquid or in solution for injection, serves as an excellent host of medically/surgically useful substances including insoluble solids such as demineralized bone powder, collagen and insoluble collagen derivatives, hydroxyapaptite, etc., and soluble solids and/or liquids dissolved therein. The above EOC can be used in many ways to promote bone growth. For example, EOC can be provided in the form a dry, flowable and/or mixable substance within a natural and/or synthetic implant.

### EXAMPLE 1 (not claimed)

### 24 hour Acid-Promoted Cleavage using Tartaric Acid pH 0.1-0.2.

In one exemplary embodiment of the present invention, the method of making EOC described above is used with the following changes. The DBM is sieved to an average particle size of from about 110 microns to about 850 microns. The DBM is then introduced into a 250mL beaker for the acid-promoted cleavage process wherein a solution of Tartaric acid with a pH of 0.15 (4.2 M) is then added to the beaker using 11 mL/g wherein g is the measurement unit for the DBM. During the acid-promoted cleavage process, the Tartaric acid solution containing the DBM is subjected to a controlled temperature of 29°C for 24 hours using an orbital mixing motion between 10 to 500 RPM (e.g., 220 RPM), preferably between 100 to 400 RPM, and more preferably between 150-250 RPM.

### EXAMPLE 2

### 48 hour Acid-Promoted Cleavage using Tartaric Acid pH 0.1-0.2.

In another exemplary embodiment of the present invention, the method of making EOC described above is used with the following changes. The DBM is sieved to an average particle size of from about 110 to about 850 microns. This DBM is then introduced into a 250mL beaker for the acid-promoted cleavage process wherein a solution of Tartaric acid with a pH of 0.15 (4.2 M) is then added to the beaker using 11 mL/g wherein g is the measurement unit for the DBM. During the acid-promoted cleavage process, the Tartaric acid solution containing the DBM is subjected to a controlled temperature of 29°C for 48 hours using an orbital mixing motion between 10 to 500 RPM (e.g., 220 RPM), preferably between 100 to 400 RPM, and more preferably between 150-250 RPM.

### EXAMPLE 3

### 72 hour Acid-Promoted Cleavage using Tartaric Acid pH 0.3-0.4.

In yet another exemplary embodiment of the present invention, the method of making EOC described above is used but with the following specifications. The DBM is sieved to an average particle size of from about 110 to about 850 microns. This DBM is then introduced into a 250mL beaker for the acid-promoted cleavage process wherein a solution of Tartaric acid with a pH of 0.35 (3.5 M) is then added to the beaker using 20 mL/g wherein g is the measurement unit for the DBM. During the acid-promoted cleavage process, the Tartaric acid solution containing the DBM is subjected to a controlled temperature of 29°C for 72 hours using an orbital mixing motion between 10 to 500 RPM (e.g., 220 RPM), preferably between 100 to 400 RPM, and more preferably between 150-250 RPM.

### EXAMPLE 4

### 96 hour Acid-Promoted Cleavage using Tartaric Acid pH 0.35-0.45.

In one exemplary embodiment of the present invention, the method of making EOC described above is used but with the following specifications. The DBM is sieved to an average particle size of from about 110 to about 850 microns. This DBM is then introduced into a 250mL beaker for the acid-promoted cleavage process wherein a solution of Tartaric acid with a pH of 0.40 (3.3 M) is then added to the beaker using 20 mL/g wherein g is the measurement unit for the DBM. During the acid-promoted cleavage process, the Tartaric acid solution containing the DBM is subjected to a controlled temperature of 29°C for 96 hours using an orbital mixing motion between 10 to 500 RPM (e.g., 220 RPM), preferably between 100 to 400 RPM, and more preferably between 150-250 RPM.

### EXAMPLE 5

### Neutralization of Osteogenic Composition

The viscous liquid composition described above in Example 3 and Example 4 is diluted to 25 mL/g using deionized water or the like and dialyzed using a Regenerated Cellulose Membrane of 3.5 kDa (pore size) against deionized water until the pH of the EOC reaches a pH range between 4.50 to 7.0, preferably between 4.75 to 7.0, and more preferably between 5.0 to 7.0. Please note that "g" (i.e., gram) is the measurement unit for the DBM originally introduced to the acid-promoted cleavage process resulting in the viscous liquid composition.

### EXAMPLE 6

### Neutralization of Osteogenic Composition

The viscous liquid composition described above in Example 3 and Example 4 is diluted to 25 mL/g using deionized water or the like and dialyzed using a Regenerated Cellulose Membrane of 6 kDa (pore size) against deionized water until the pH range between 4.50 to 7.0, preferably between 4.75 to 7.0, and more preferably between 5.0 to 7.0.

### EXAMPLE 7

### Neutralization of Osteogenic Composition

The viscous liquid composition described above in Example 1, 2, 3 and 4 is directly dialyzed (i.e., undiluted) using a Regenerated Cellulose Membrane of 3.5 kDa (pore size) against deionized water until the pH range between 4.50 to 7.0, preferably between 4.75 to 7.0, and more preferably between 5.0 to 7.0.

### EXAMPLE 8

### Neutralization of Osteogenic Composition

The viscous liquid composition described above in Example 1, 2, 3 and 4 is directly dialyzed (i.e., undiluted) using a Regenerated Cellulose Membrane of 6 kDa (pore size) against deionized water until the pH range between 4.50 to 7.0, preferably between 4.75 to 7.0, and more preferably between 5.0 to 7.0.

### EXAMPLE 9

### Dry Osteoinductive and Osteoconductive Granule

The EOC described above in Example 5, 6, 7 and 8 still in viscous liquid form would be mixed with a synthetic calcium phosphate powder, by undergoing a solid state reaction, and this mixture will turn into a solid, which will then be grinded down to a granule. This granule could be in the form of an Apatite with a porosity of 30% to 90 %, with a granules size of 500 to 700 micron.

### EXAMPLE 10

### Osteoinductive and Osteoconductive Flowable Bone Repair Composition

The EOC described above in Example 5, 6, 7 and 8 still in a viscous liquid form would be mixed through a carrier of nano-sized calcium phosphate particles suspended in an aqueous solution.

### EXAMPLE 11

### Dry EOC Particles

The EOC described above in Example 5, 6, 7 and 8 still in a viscous liquid form is casted in a PETG tray with or without wells or other suitable casting materials ranging from 1-2500 mL in volume. Once casted, the casting trays or similar are placed in a deep freeze at about -40°C to -80°C for a minimum of 24 hours, then lyophilized over multiple days to remove excess moisture to form a lyophilized dried EOC. This lyophilized EOC can be blended into small particles to create a "pixie dust" like product having an average particle size of between 0.1 microns to 2000 micron ("EOC Particles" and use for surgical implantation or mixed with additional active or inactive fillers.

Additionally, cancellous bone and/or demineralized bone matrix/powder may also be added, either alone as particles or in combination with one another, to the dried EOC Particles. After terminal sterilization, this material may be used for insertion at, in, on, or near a bone and/or chondral defects.

In addition, the lyophilized dried EOC Particles or prior to blending into particulate form can be hydrated using deionized water or buffer saline at a concentration up to 10.0 mL/g. This viscous mixture is then poured in a tray with the desired shape and volume. The above freezing and lyophilization steps are repeated. This composition, before or after hydration, may be used for injection or surgical implantation at bone defect sites or mixed with additional active or inactive fillers i.e., collagen sponge, demineralized bone matrix/powder, CaP, etc.

### EXAMPLE 12

### Dry EOC Matrix Booster

The EOC described above in Example 5, 6, 7 and 8 still in a viscous liquid form, prior to the freezing step described above, can be mixed with cancellous chips, demineralized bone fibers, cancellous sponge, demineralized bone matrix chips and demineralized bone matrix granules. The combined product is then frozen and lyophilized. These materials may be used for surgical implantation at, in, on, or near the bone and/or chondral defect sites.

### EXAMPLE 13

### Dry Fibrous EOC Matrix with Instant imbibition

The EOC described above in Example 5, 6, 7 and 8 still in a viscous liquid form is casted in a Polypropylene (PP) Cup up to 50mL then placed inside an aluminum alloy lyophilization tray with Tyvek cover (lid). The lyophilization tray is then placed in a deep freeze at about -40°C to -80°C for a minimum of 24 hours, and then lyophilized over multiple days to remove excess moisture to form a lyophilized dry fibrous matrix ("Fibrous EOC Matrix"). After terminal sterilization, using gamma or ebeam (or similar methods), the lyophilized Fibrous EOC Matrix can be hydrated using a buffered saline, saline, blood, bone marrow then mixed with additional active or inactive fillers i.e., collagen sponge, demineralized bone matrix/powder, CaP, etc. These materials may be used for surgical implantation at, in, on, or near the bone and/or chondral defect sites. The Fibrous EOC Matrix has instant imbibition (absorption) properties defined as having the ability to absorb in less than 60 seconds (i.e., having a range of from 1 second to 60 seconds, preferably from 5 seconds to 30 seconds, and more preferably from 10 seconds to 20 seconds).

### EXAMPLE 14

### Histopathology

In this study, the viscous liquid composition described above in Example 2 is diluted to 25 mL/g using deionized water or the like and dialyzed using a Regenerated Cellulose Membrane of 3.5 kDa (pore size) against deionized water until the pH of the EOC reaches a pH range between 4.50 to 7.0, preferably between 4.75 to 7.0, and more preferably between 5.0 to 7.0. Please note that "g" (i.e., gram) is the measurement unit for the DBM originally introduced to the acid-promoted cleavage process resulting in the viscous liquid composition.

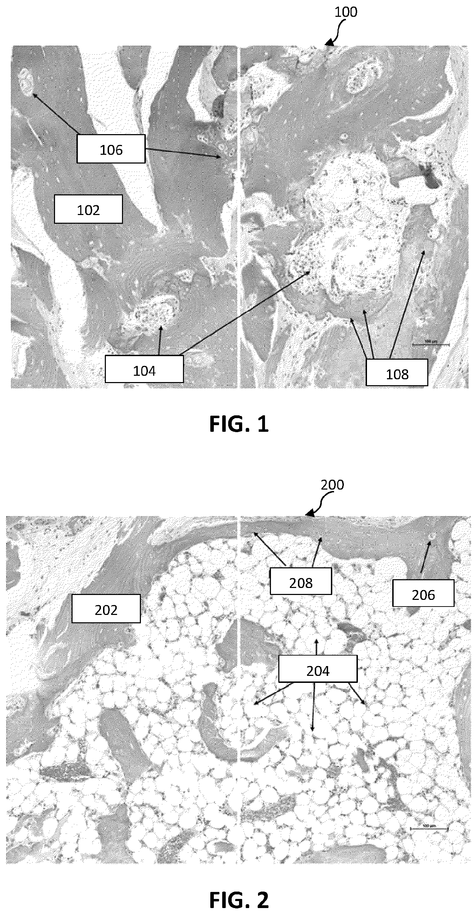

DBM is also used in this study. Approximately 0.1 gram of the EOC described above in this example is combined with approximately 0.1 gram of DBM ("EOC Sample") is then implanted into each of the *biceps femoris* muscles of eight (8) athymic male rats ("animal"). For control purposes, approximately 0.2 gram of DBM (from the same lot/source as the DBM used in the EOC Samples) was also implanted into the left biceps femoris muscles of four (4) animals. During implantation, each animal was anesthetized and prepared for surgery. A pocket was created in each biceps femoris muscle using sharp and blunt dissection. After the incision was made, either the control DBM sample or the EOC Sample was placed into the muscle pocket, and then the muscle pocket and skin were sutured closed. After the surgery, the animals were observed daily for their general health status for twenty-eight (28) days. During this period, the animals gained weight and no abnormal clinical signs were noted for any of the animals. After twenty-eight (28) days, the animals were sacrificed when all implants sites were positively identified and harvested as study samples. Evaluation of these study samples showed that the DBM only study samples (sites implanted with 0.2 gram of DBM only) scored on a "1" for osteoinductivity, which is defined as 0 to 25% new bone formation as shown in FIG. 1. Referring to FIG. 1, which is is a representative histopathology photograph of one DBM only study sample 100 taken at 100X total magnification showing the EOC 102, the bone marrow 104, the cartilage 106, and the new bone formation 108 of the sample 100 implant site.

The EOC study samples (sites implanted with 0.1 gram of EOC and 0.1 gram of DBM) scored a "4" for osteoinductivity, which is defined as 75% to 100% new bone formation as shown in FIG. 2. Referring to FIG. 2 which is is a representative histopathology photograph of one EOC study sample 200 (out of the sixteen samples) taken at 100X total magnification showing the EOC 202, the bone marrow 204, the cartilage 206, and the new bone formation 208 of the sample 200 implant site. The study's results demonstrated osteoinduction potential of the EOC in the intramuscular implant sites using the male athymic nude rat model. It should be noted that while the absolute scores of the study samples may vary from animal to animal, the study clearly indicates the EOC study samples met the histological criteria for evidence of osteoinduction as elements of new bone formation were observed in all sixteen samples and had an increase of new bone formation when compared to the control DBM.

## Claims

1. A method for preparing an enhanced osteogenic composition comprising the step of treating demineralized bone material in an organic acidic extraction medium in a concentration of at least about 3.0 M to about 5.0 M at an extraction temperature between greater than 25° C and less than 80° C for a predetermined time period of between 48 hours to 120 hours, wherein:
a) the enhanced osteogenic composition, derived from the demineralized bone material treated in the organic acidic extraction medium, comprises connective tissue proteins with molecular weights greater than or equal to 3.5 kDa,
wherein the organic acidic extraction medium is tartaric acid.

2. Method according to claim 1 wherein the organic acidic extraction medium has a ratio of acid to the demineralized bone material of at least about 1mL/g to about 100mL/g.

3. Method according to the preceding claims wherein the demineralized bone material has an average particle size from about 110 microns to about 850 microns.

4. Method according to the preceding claims wherein the extraction temperature is between 26° C and 50 °C.

5. Method according to the preceding claims wherein the extraction temperature is between 26° C and 37° C.

6. Method according to the preceding claims wherein the enhanced osteogenic composition is further prepared by one of more of the following steps:
- titration and the composition has a pH greater than or equal to 4.5;
- dialyzing the viscous liquid composition with a Regenerated Cellulose Membrane having a pore size greater than or equal to 3.5 kDa resulting in the enhanced osteogenic composition having a pH greater than or equal to 4.5;
- dialysis and lyophilization.

7. Method according to claim 6 further comprising the steps
- mixing the enhanced osteogenic composition in a viscous liquid form with a synthetic calcium phosphate powder resulting in a mixture;
- grinding the mixture to form solid granules.

8. Method according to claim 7 wherein the mixing step involves
- mixing the enhanced osteogenic composition in a viscous liquid form with a carrier comprising of nano-sized calcium phosphate particles suspended in an aqueous solution to create a flowable bone repair composition.

9. Enhanced osteogenic composition obtainable by the method according to claims 1 to 6.

10. Enhanced osteogenic composition according to claim 9 wherein the composition is in the form of a lyophilized dry fibrous matrix.

11. Enhanced osteogenic composition according to claims 9 and 10 wherein the enhanced osteogenic composition in a viscous liquid form is casted, frozen, lyophilized, and blended into small pixie dust particles having an average particle size of between 0.1 micron to 2000 micron.

12. Flowable bone repair composition obtainable by the process of claim 8.

13. Enhanced osteogenic composition according to claims 9 - 11 and flowable bone repair composition according to claim 12 for use in promoting bone growth.

## Patentansprüche

1. Verfahren zur Herstellung einer verbesserten osteogenen Zusammensetzung, das den Schritt des Behandelns von demineralisiertem Knochenmaterial in einem organischen sauren Extraktionsmedium in einer Konzentration von mindestens etwa 3,0 M bis etwa 5,0 M bei einer Extraktionstemperatur zwischen mehr als 25 °C und weniger als 80 °C für einen vorbestimmten Zeitraum zwischen 48 Stunden bis 120 Stunden umfasst, wobei:
a) die verbesserte osteogene Zusammensetzung, die aus dem in dem organischen sauren Extraktionsmedium behandelten demineralisierten Knochenmaterial gewonnen wird, Bindegewebsproteine mit Molekulargewichten umfasst, die größer als oder gleich 3,5 kDa sind,
wobei das organische saure Extraktionsmedium Weinsäure ist.

2. Verfahren nach Anspruch 1, wobei das organische saure Extraktionsmedium ein Verhältnis von Säure zu demineralisiertem Knochenmaterial von mindestens etwa 1 mL/g bis etwa 100 mL/g aufweist.

3. Verfahren nach den vorhergehenden Ansprüchen, wobei das demineralisierte Knochenmaterial eine durchschnittliche Partikelgröße von etwa 110 Mikron bis etwa 850 Mikron aufweist.

4. Verfahren nach den vorhergehenden Ansprüchen, wobei die Extraktionstemperatur zwischen 26 °C und 50 °C liegt.

5. Verfahren nach den vorhergehenden Ansprüchen, wobei die Extraktionstemperatur zwischen 26 °C und 37 °C liegt.

6. Verfahren nach den vorhergehenden Ansprüchen, wobei die verbesserte osteogene Zusammensetzung ferner durch einen oder mehrere der folgenden Schritte hergestellt wird:
- Titration und die Zusammensetzung weist einen pH-Wert auf, der größer als oder gleich 4,5 ist;
- Dialysieren der viskosen Flüssigkeitszusammensetzung mit einer regenerierten Zellulosemembran mit einer Porengröße, die größer als oder gleich 3,5 kDa ist, was zu der verbesserten osteogenen Zusammensetzung mit einem pH-Wert führt, der größer als oder gleich 4,5 ist;
- Dialyse und Lyophilisierung.

7. Verfahren nach Anspruch 6, das ferner die folgenden Schritte umfasst:
- Mischen der verbesserten osteogenen Zusammensetzung in einer viskosen flüssigen Form mit einem synthetischen Calciumphosphatpulver, was zu einer Mischung führt;
- Vermahlen der Mischung, um feste Granulate zu bilden.

8. Verfahren nach Anspruch 7, wobei der Mischschritt Folgendes umfasst:
- Mischen der verbesserten osteogenen Zusammensetzung in einer viskosen flüssigen Form mit einem Träger, der aus in einer wässrigen Lösung suspendierten nanoskaligen Calciumphosphatpartikeln besteht, um eine fließfähige Knochenreparaturzusammensetzung herzustellen.

9. Verbesserte osteogene Zusammensetzung, die durch das Verfahren nach den Ansprüchen 1 bis 6 erhältlich ist.

10. Verbesserte osteogene Zusammensetzung nach Anspruch 9, wobei die Zusammensetzung in Form einer lyophilisierten, trockenen Fasermatrix ist.

11. Verbesserte osteogene Zusammensetzung nach den Ansprüchen 9 und 10, wobei die verbesserte osteogene Zusammensetzung in einer viskosen flüssigen Form gegossen, gefroren, lyophilisiert und zu kleinen Feenstaubpartikeln mit einer durchschnittlichen Partikelgröße zwischen 0,1 Mikron und 2000 Mikron vermischt wird.

12. Fließfähige Knochenreparaturzusammensetzung, die durch das Verfahren nach Anspruch 8 erhältlich ist.

13. Verbesserte osteogene Zusammensetzung nach den Ansprüchen 9 - 11 und fließfähige Knochenreparaturzusammensetzung nach Anspruch 12 zur Verwendung bei der Förderung des Knochenwachstums.

## Revendications

1. Procédé de préparation d'une composition ostéogénique améliorée comprenant l'étape de traitement d'un matériau osseux déminéralisé dans un milieu d'extraction d'acide organique à une concentration d'au moins environ 3,0 M à environ 5,0 M à une température d'extraction comprise entre plus de 25 °C et moins de 80 °C pendant une période de temps prédéterminée comprise entre 48 heures et 120 heures, où :
a) la composition ostéogénique améliorée, dérivée du matériau osseux déminéralisé traité dans le milieu d'extraction d'acide organique, comprend des protéines de tissu conjonctif ayant des poids moléculaires supérieurs ou égaux à 3,5 kDa,
où le milieu d'extraction d'acide organique est de l'acide tartrique.

2. Procédé selon la revendication 1, où le milieu d'extraction d'acide organique a un rapport d'acide sur matériau osseux déminéralisé d'au moins environ 1 ml/g à environ 100 ml/g.

3. Procédé selon les revendications précédentes, où le matériau osseux déminéralisé a une taille moyenne de particules d'environ 110 microns à environ 850 microns.

4. Procédé selon les revendications précédentes, où la température d'extraction est comprise entre 26 °C et 50 °C.

5. Procédé selon les revendications précédentes, où la température d'extraction est comprise entre 26 °C et 37 °C.

6. Procédé selon les revendications précédentes, où la composition ostéogénique améliorée est en outre préparée par une ou plusieurs des étapes suivantes :
- titrage et la composition a un pH supérieur ou égal à 4,5 ;
- dialyser la composition liquide visqueuse à l'aide d'une membrane en cellulose régénérée ayant une taille de pores supérieure ou égale à 3,5 kDa résultant en la composition ostéogénique améliorée ayant un pH supérieur ou égal à 4,5 ;
- dialyse et lyophilisation.

7. Procédé selon la revendication 6, comprenant en outre les étapes suivantes :
- mélanger la composition ostéogénique améliorée sous forme de liquide visqueux avec une poudre de phosphate de calcium synthétique résultant en un mélange ;
- broyer le mélange pour former des granules solides.

8. Procédé selon la revendication 7, où l'étape de mélange implique
- mélanger la composition ostéogénique améliorée sous forme de liquide visqueux avec un support comprenant des particules de phosphate de calcium de taille nanométrique en suspension dans une solution aqueuse pour créer une composition fluide de réparation osseuse.

9. Composition ostéogénique améliorée pouvant être obtenue par le procédé selon les revendications 1 à 6.

10. Composition ostéogénique améliorée selon la revendication 9, où la composition se présente sous la forme d'une matrice fibreuse sèche lyophilisée.

11. Composition ostéogénique améliorée selon les revendications 9 et 10, où la composition ostéogénique améliorée sous forme de liquide visqueux est coulée, congelée, lyophilisée et mélangée en petites particules de type « poussière de fée » ayant une taille moyenne de particule comprise entre 0,1 micron et 2000 microns.

12. Composition fluide de réparation osseuse pouvant être obtenue par le processus selon la revendication 8.

13. Composition ostéogénique améliorée selon les revendications 9 à 11 et composition fluide de réparation osseuse selon la revendication 12 pour une utilisation pour favoriser la croissance osseuse.
